# EUROPEAN PATENT APPLICATION

(11) **EP 4 834 316 A1**
(43) Date of publication of application: **04.11.2026**
(21) Application number: 24912692.1
(22) Date of filing: 23.12.2024
(51) Int. Cl.: A61K 8/365, A61K 8/04, A61K 8/19, A61K 8/37

(54) **METAL OXIDE DISPERSION COMPOSITION**

(30) Priority: 27.12.2023 JP 2023221040
(71) Applicant: KOSE Holdings Corporation, Tokyo 103-8251 (JP)
(72) Inventor: SASAKI, Yudai, Tokyo 114-0005 (JP); TAKATA, Ami, Tokyo 114-0005 (JP); IO, Yuto, Tokyo 114-0005 (JP); Ohtani, Kouhei, Tokyo 114-0005 (JP)
(74) Representative: D Young & Co LLP
(86) International application number: PCT/JP2024/045342
(87) International publication number: WO 2025/142805

(57) **Abstract**

Provided is a metal oxide dispersion composition having excellent long-term stability, excelling in no stickiness, and achieving good usability. The metal oxide dispersion composition comprises components (A) to (C): (A) a polyhydroxystearic acid, (B) a silicone-treated metal oxide, and (C) a glyceryl oleate, and the metal oxide in the component (B) is a metal oxide excluding titanium oxide.

## Description

### Technical Field

The present invention relates to a metal oxide dispersion composition and specifically relates to a metal oxide dispersion composition having excellent long-term stability, no stickiness, and good usability.

### Background Art

Metal oxides contained in cosmetics, paints, and the like are used as raw materials having various functions. In particular, these metal oxides have such characteristics as to achieve various effects depending on the size (average particle size). A metal oxide having a large average particle size may be used as a white pigment for a colorant, while a metal oxide having a small average particle size features excellent UV protection and is used in sunscreen cosmetics and similar products. A metal oxide having a smaller average particle size is, however, likely to have lower dispersibility in a composition and may tend to form aggregates with each other.

To solve such problems, a technique of surface-treating a metal oxide has been disclosed to increase the degree of hydrophobicity and to improve the dispersibility. For example, a technique for improving the dispersibility by surface treatment with a solid compound and a liquid compound at normal temperature (for example, see Patent Document 1) and a technique for improving the dispersibility by surface treatment with an alkylalkoxysilane (see Patent Document 2) are disclosed.

### Citation List

### Patent Documents

Patent Document 1: JP-A No. 2001-72527
Patent Document 2: JP-A No. Hei-8-119832

### Summary of the Invention

### Technical Problem

In the technique disclosed in Patent Document 1, a metal oxide surface-treated with a solid compound has poor smoothness when applied onto the skin and results in poor spreadability. In the technique disclosed in Patent Document 2, an oil phase is required to contain a large amount of silicone oil, and this limits formulation flexibility. For example, an oily agent having poor compatibility, such as a solid ultraviolet absorber, is difficult to contain. The techniques disclosed in Patent Document 1 and Patent Document 2 may cause gelation over time and thus are unable to provide a composition that causes no gelation over time, has excellent long-term stability, excels in no stickiness, and achieves good usability.

A metal oxide dispersion composition of the present invention has excellent long-term stability, excels in no stickiness, and achieves good usability.

### Solution to Problem

In such circumstances, the inventors of the present invention have conducted intensive studies to solve the problems, have found that combination use of a silicone-treated metal oxide, a polyhydroxystearic acid, and a glyceryl oleate enables production of a metal oxide dispersion composition having excellent long-term stability and excelling in no stickiness, and have accomplished the present invention.

The present invention provides a metal oxide dispersion composition comprising components (A) to (C):
(A) a polyhydroxystearic acid,
(B) a silicone-treated metal oxide, and
(C) a glyceryl oleate,
wherein the metal oxide in the component (B) is a metal oxide excluding titanium oxide.

The mass ratio of the component (B) relative to the total content of the component (A) and the component (C), (B)/{(A) + (C)}, may be less than 12.

The component (B) may be a dimethylpolysiloxane-treated metal oxide.

The content of the component (B) may be 20 to 80% by mass.

The mass ratio of the component (B) relative to the component (C), (B)/(C), may be 1 to 30.

The mass ratio of the component (A) relative to the component (C), (A)/(C), may be 0.1 to 3.0.

The metal oxide in the component (B) may be zinc oxide.

The metal oxide dispersion composition may further comprise a component (D), a polyhydric alcohol.

The present invention also provides a cosmetic or a topical skin preparation comprising the metal oxide dispersion composition.

The cosmetic or the topical skin preparation may be an oil-in-water emulsion.

The cosmetic or the topical skin preparation may be used for sun protection.

The present invention also provides a cosmetic or a topical skin preparation comprising components (A) to (C):
(A) a polyhydroxystearic acid,
(B) a silicone-treated metal oxide, and
(C) a glyceryl oleate,
wherein the metal oxide in the component (B) is a metal oxide excluding titanium oxide.

In the cosmetic or the topical skin preparation, the mass ratio of the component (B) relative to the total content of the component (A) and the component (C), (B)/{(A) + (C)}, may be less than 12.

In the cosmetic or the topical skin preparation, the component (B) may be a dimethylpolysiloxane-treated metal oxide.

In the cosmetic or the topical skin preparation, the content of the component (B) may be 1 to 35% by mass.

In the cosmetic or the topical skin preparation, the mass ratio of the component (B) relative to the component (C), (B)/(C), may be 1 to 30.

In the cosmetic or the topical skin preparation, the mass ratio of the component (A) relative to the component (C), (A)/(C), may be 0.1 to 3.0.

In the cosmetic or the topical skin preparation, the metal oxide in the component (B) may be zinc oxide.

The cosmetic or the topical skin preparation may further comprise a component (D), a polyhydric alcohol.

In the cosmetic or the topical skin preparation, the component (D) may be glycerol.

The cosmetic or the topical skin preparation may be an oil-in-water emulsion.

The cosmetic or the topical skin preparation may be used for sun protection.

### Advantageous Effects of Invention

A metal oxide dispersion composition of the present invention has excellent long-term stability, excels in no stickiness, and achieves good usability.

The metal oxide dispersion composition of the present invention comprises a polyhydroxystearic acid as a component (A) and a glyceryl oleate as a component (C) in combination. Hence, the composition has excellent long-term stability, excels in no stickiness, and achieves good usability even when comprising a silicone-treated metal oxide, which is surface-treated by physical adsorption giving lower adsorption power than that by chemical adsorption.

### Brief Description of Drawings

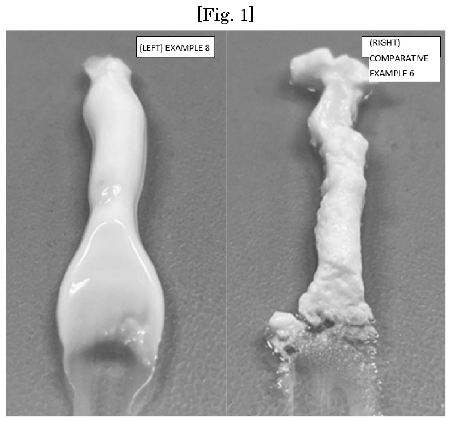

Fig. 1 is a photograph of appearances of oil-in-water emulsion cosmetics in Example 8 (left) and Comparative Example 6 (right).

### Description of Embodiments

The present invention will now be described in detail. In the present description, "to" between numerical values denotes a range including the numerical values. A numerical value with % is on the basis of mass, unless otherwise specified. The embodiments described below are merely typical embodiments of the present invention, and the scope of the present invention is not limited to these embodiments.

The present invention will be described in the following order.
1. First embodiment
   (1) Formulation of metal oxide dispersion composition
   (2) Components
   (3) Production method of metal oxide dispersion composition
   (4) Application
2. Second embodiment
   (1) Formulation of cosmetic
   (2) Components
   (3) Production method of cosmetic
   (4) Application
3. Third embodiment
   (1) Formulation of topical skin preparation
   (2) Components
   (3) Production method of topical skin preparation
   (4) Application
4. Fourth embodiment
   (1) Formulation of cosmetic
   (2) Components
   (3) Production method of cosmetic
   (4) Application
5. Fifth embodiment
   (1) Formulation of topical skin preparation
   (2) Components
   (3) Production method of topical skin preparation
   (4) Application
6. Examples

In the present description, measurements are conducted under conditions of 25°C ± 2°C and 50% RH ± 5% RH, unless the measurement environment is specifically stated in the description of measurement methods.

### 1. First embodiment

### (1) Formulation of metal oxide dispersion composition

A metal oxide dispersion composition of the present embodiment comprises a polyhydroxystearic acid as a component (A), a silicone-treated metal oxide as a component (B), and a glyceryl oleate as a component (C), and the metal oxide in the component (B) is a metal oxide excluding titanium oxide. The components contained in the metal oxide dispersion composition will next be described.

### (2) Components

### A. [Component (A): polyhydroxystearic acid]

The polyhydroxystearic acid as the component (A) in the embodiment is a polymer of a hydroxystearic acid having a hydroxy group. The polyhydroxystearic acid as the component (A) is used to disperse the silicone-treated metal oxide as the component (B). The hydroxy group of the hydroxystearic acid is preferably located at the 12-position, and the polymerization degree of the hydroxystearic acid is preferably 3 to 12 and more preferably 4 to 8. Examples of the commercial product include SALACOS HS-6C (manufactured by The Nisshin OilliO Group, Ltd.) and ARLACEL P-100 (manufactured by Uniqema).

### B. [Component (B): silicone-treated metal oxide]

In the silicone-treated metal oxide as the component (B) in the embodiment, the metal oxide is a metal oxide that, for example, absorbs, scatters, reflects, or quenches ultraviolet rays in a wide range from UVB to UVA (290 to 400 nm) to block ultraviolet rays and excludes titanium oxide, and may be any metal oxide that is commonly used in cosmetics, quasi-drugs, pharmaceutical products, or similar products. The metal oxide to be used is preferably at least one selected from the group consisting of zinc oxide, cerium oxide, iron oxide, and chromium oxide from the viewpoint of easy availability on the market. From the viewpoint of ultraviolet ray protection effects or similar effects, at least one selected from the group consisting of zinc oxide and cerium oxide is more preferably used, and zinc oxide is particularly preferably used. The metal oxide may contain a trace element having a valence of +2 or higher, and the metal oxide may contain a single of or an appropriate combination of two or more of metals such as iron, zirconium, calcium, manganese, magnesium, and yttrium.

The metal oxide may have any particle size. From the viewpoint of ultraviolet ray protection effects or similar effects, the average particle size is preferably 1 nm to 1 µm, more preferably 3 nm to 100 nm, and even more preferably 5 nm to 50 nm.

In the present embodiment, the average particle size is determined by image analysis of a transmission electron microscope image. The measurement method of the average particle size will next be described.

On a glass slide, about 10 mg of a sample is kneaded with 1-propanol and well dispersed to give a dispersed sample. The dispersed sample is spread to give a thin film sample. The thin film sample is placed on a transmission electron microscope (TEM) measurement grid coated with a support film. The grid (dried coating) is mounted in a transmission electron microscope (S-4800, Hitachi High-Technologies Corporation) and is observed to prepare an image of the dried coating film showing individual particles. The dried coating film image is subjected to image processing with an image-based particle size distribution analyzer (Mac-View, Mauntech Co., Ltd.) to determine the particle sizes of 1,000 particles.

As a result, the image analysis of a transmission electron microscope (TEM) image provides the average particle size D50 of the sample (powder).

The metal oxide may have any shape. Examples of the shape include a granular shape, a spherical shape, a plate shape, a spindle shape, a dendritic shape, and a balloon shape. From the viewpoint of ultraviolet ray protection effects or similar effects, the shape is preferably a granular shape, a spherical shape, a plate shape, or a spindle shape.

The metal oxide is not specifically limited. As zinc oxide, for example, FINEX-25 (manufactured by Sakai Chemical Industry Co., Ltd.), FINEX-50 (manufactured by Sakai Chemical Industry Co., Ltd.), FINEX-75 (manufactured by Sakai Chemical Industry Co., Ltd.), MZ300 series (manufactured by Tayca Corporation), MZ500 series (manufactured by Tayca Corporation), MZ700 series (manufactured by Tayca Corporation), and ZnO-350 (manufactured by Sumitomo Osaka Cement Co., Ltd.) are commercially available. As cerium oxide, for example, high-purity cerium is commercially available from NIKKI CORPORATION or Seimi Chemicals Co., Ltd.

The silicone-treated metal oxide is prepared by subjecting the metal oxide to silicone treatment. Examples of the silicone treatment include a treatment with a silicone treatment agent. Examples of the silicone treatment agent include chain silicones such as low-polymerization-degree dimethylpolysiloxane, high-polymerization-degree dimethylpolysiloxane, and methylphenylpolysiloxane; modified silicones such as amino-modified silicone, alkyl-modified silicone, and alkoxy-modified silicone; silicone resins such as trimethylsiloxy silicate and acrylic-silicone graft copolymers; silicone rubbers; partially or fully cross-linked organopolysiloxanes; silylation agents; and silane coupling agents. At least one selected from these groups may be used. In the present embodiment, silicone treatment by physical adsorption giving lower adsorption power than that by chemical adsorption is preferred, and treatment with dimethylpolysiloxane is more preferred.

The silicone treatment may be performed by any method, and a conventionally known method may be used. For example, a silicone treatment agent and metal oxide particles as a powder to be treated are added to a solvent; the mixture is stirred with a ball mill or the like; the resulting mixture is dried as needed and is repeatedly washed with water and filtered to remove impurities; and the product is dried and pulverized to give an intended silicone-treated metal oxide powder. Several types of metal oxides may be simultaneously subjected to silicone treatment. Alternatively, any one of metal oxides may be previously subjected to silicone treatment, and then the other metal oxides may be subjected to silicone treatment. The amount of a silicone treatment agent relative to the metal oxide is not necessarily limited but is preferably 40% or less and more preferably 2 to 35%.

### C. [Component (C): glyceryl oleate]

The glyceryl oleate as the component (C) used in the present embodiment may be at least one selected from the group consisting of glyceryl monooleate, glyceryl dioleate, and glyceryl trioleate. Specifically, the glyceryl oleate preferably comprises glyceryl monooleate and is more preferably glyceryl monooleate.

The glyceryl oleate is not specifically limited. For example, NIKKOL MGO (product name, manufactured by Nikko Chemicals Co., Ltd.) and GLYMOIST MO (manufactured by NOF CORPORATION) are commercially available.

### D. [Component (D): polyhydric alcohol]

The metal oxide dispersion composition of the present embodiment may further comprise a component (D), a polyhydric alcohol. Examples of the polyhydric alcohol include, but are not necessarily limited to, glyceryl, glycerol, 1,3-butylene glycol, dipropylene glycol, tripropylene glycol, and sugar alcohols such as sorbitol and mannitol. The polyhydric alcohol is particularly preferably glyceryl.

### E. [Additional components]

The metal oxide dispersion composition of the present embodiment may comprise, in addition to the above components, powders other than the above, a surfactant, an oil medium, a gelling agent, an antioxidant, an antiseptic, a colorant, a flavoring agent, a cosmetic ingredient, and other components, within a quantitative and qualitative range that does not impair the effects of the present embodiment, depending on the purpose. Further details will be described in a second embodiment.

The metal oxide dispersion composition of the present embodiment is preferably an oily dispersion in which a powder is mainly dispersed in an oil medium. The dispersion medium may be any oil medium that is liquid at 25°C and is preferably an ester oil. Further details will be described in the second embodiment. The content of the dispersion medium is not specifically limited, but is, in the composition, preferably 5 to 70% by mass (hereinafter abbreviated as "%"), more preferably 10 to 60%, even more preferably 15 to 60%, and particularly preferably 15 to 50%. The medium that is liquid at 25°C has flowability at 25°C.

In the present embodiment, the content of the component (A) is preferably 1 to 15% by mass (hereinafter abbreviated as "%"), more preferably 2 to 10%, even more preferably 2 to 9%, furthermore preferably 3 to 9%, and particularly preferably 4 to 9%. Within the range, even better long-term stability and good usability without stickiness are achieved.

In the present embodiment, the mass ratio of the component (B) relative to the total content of the component (A) and the component (C), (B)/{(A) + (C)}, is preferably less than 12, more preferably 10 or less, even more preferably 9 or less, furthermore preferably 8 or less, particularly preferably 7 or less, and especially preferably 6 or less. Within the range, even better usability without stickiness is achieved, and the long-term stability is improved. In the present embodiment, the lower limit of the mass ratio, (B)/{(A) + (C)}, is not specifically limited because the total amount of the component (A) and the component (C) is excess relative to the component (B), and the stability is improved, but is typically preferably 1 or more, more preferably 2 or more, even more preferably 3 or more, furthermore preferably 3.5 or more, and particularly preferably 3.8 or more.

In the present embodiment, the content of the component (B) is preferably 20 to 80% by mass (hereinafter abbreviated as "%"), more preferably 30 to 80%, even more preferably 40 to 80%, and furthermore preferably 40 to 70%. Within the range, even better long-term stability and good usability without stickiness are achieved.

In the present embodiment, the content of the component (C) is preferably 1 to 15% by mass (hereinafter abbreviated as "%"), more preferably 2 to 13%, even more preferably 2.5 to 12%, furthermore preferably 3 to 12%, and particularly preferably 3 to 10%. Within the range, even better long-term stability and good usability without stickiness are achieved.

In the present embodiment, the mass ratio of the component (B) relative to the component (C), (B)/(C), is preferably 1 to 30, more preferably 2 to 28, even more preferably 4 to 25, furthermore preferably 6 to 20, particularly preferably 6 to 16, and especially preferably 6 to 15. Within the range, even better long-term stability and good usability without stickiness are achieved.

In the present embodiment, the mass ratio of the component (A) relative to the component (C), (A)/(C), is preferably 0.1 to 3.0, more preferably 0.2 to 2.8, even more preferably 0.3 to 2.5, furthermore preferably 0.4 to 2.4, and particularly preferably 0.5 to 2.0. Within the range, even better long-term stability and good usability without stickiness are achieved.

In the present embodiment, the content of the component (D) is preferably 0.1 to 5% by mass (hereinafter abbreviated as "%"), more preferably 0.3 to 4%, even more preferably 0.5 to 3%, and furthermore preferably 0.5 to 2%. Within the range, even better long-term stability and good usability without stickiness are achieved.

### (3) Production method of metal oxide dispersion composition

The metal oxide dispersion composition of the present embodiment may be produced by any method: for example, the component (A), the component (C), and optional components are mixed; the component (B) is added to the mixture; and the whole is homogeneously dispersed to give the composition. The optional components include the component (D) and an oil medium, for example. Examples of the apparatus for the homogeneous dispersion include a disper-mixer, a homomixer, a roll mill, a ball mill, and a bead mill. A wet milling apparatus using a medium, such as a roll mill, a ball mill, and a bead mill, is more preferably used.

### (4) Application

The metal oxide dispersion composition of the present embodiment is applicable to cosmetics as well as pharmaceutical products such as a topical skin preparation, paints, inks, optical filters, and laminated paper.

### 2. Second embodiment

### (1) Formulation of cosmetic

A cosmetic of the present embodiment comprises the metal oxide dispersion composition in the first embodiment. In the cosmetic of the present embodiment, the content of the metal oxide dispersion composition is preferably 5 to 40%, more preferably 10 to 30%, even more preferably 10 to 25%, and furthermore preferably 10 to 20%. In other words, the cosmetic of the present embodiment comprises a polyhydroxystearic acid as a component (A), a silicone-treated metal oxide as a component (B), and a glyceryl oleate as a component (C), and the metal oxide in the component (B) is a metal oxide excluding titanium oxide. The components contained in the cosmetic will next be described.

### (2) Components

As for the polyhydroxystearic acid as the component (A), the silicone-treated metal oxide as the component (B), the glyceryl oleate as the component (C), and the polyhydric alcohol as the component (D), the above description in the first embodiment also applies to the present embodiment, and a detailed explanation is omitted.

The cosmetic of the present embodiment may appropriately comprise, within the range not impairing the effects of the present embodiment, in addition to the above components, components typically contained in cosmetics, including water, an oil medium, powders such as an inorganic pigment, an organic pigment, and an extender pigment, a surfactant, aqueous components such as a lower alcohol, a water-soluble polymer, and a moisturizer, a sugar, an ultraviolet absorber, an antioxidant, an anti-fading agent, an antiseptic, a pharmaceutical ingredient, a stabilizer, a pigment, and a flavoring agent, as needed, for imparting various effects. Within a range not impairing the effects of the present embodiment, in place of a metal dispersion composition comprising the above components, the respective components may be separately added while the components are not added to prepare a metal oxide dispersion composition, as needed.

In the cosmetic of the present embodiment, the content of the component (A) is preferably 0.05 to 6% by mass (hereinafter abbreviated as "%"), more preferably 0.1 to 4%, even more preferably 0.2 to 3%, furthermore preferably 0.4 to 2.5%, and particularly preferably 0.5 to 2%. Within the range, even better long-term stability and good usability without stickiness are achieved.

In the cosmetic of the present embodiment, the mass ratio of the component (B) relative to the total content of the component (A) and the component (C), (B)/{(A) + (C)}, is preferably less than 12, more preferably 10 or less, even more preferably 9 or less, furthermore preferably 8 or less, particularly preferably 7 or less, and especially preferably 6 or less. Within the range, even better usability without stickiness is achieved, and the long-term stability is improved. In the present embodiment, the lower limit of the mass ratio, (B)/{(A) + (C)}, is not specifically limited because the total amount of the component (A) and the component (C) is excess relative to the component (B), and the stability is improved, but is typically preferably 1 or more, more preferably 2 or more, even more preferably 3 or more, furthermore preferably 3.5 or more, and particularly preferably 3.8 or more.

In the cosmetic of the present embodiment, the content of the component (B) is preferably 1 to 35% by mass (hereinafter abbreviated as "%"), more preferably 1 to 30%, even more preferably 2 to 28%, furthermore preferably 4 to 20%, and particularly preferably 4 to 15%. Within the range, even better long-term stability and good usability without stickiness are achieved.

In the cosmetic of the present embodiment, the content of the component (C) is preferably 0.05 to 6% by mass (hereinafter abbreviated as "%"), more preferably 0.1 to 4%, even more preferably 0.15 to 3%, furthermore preferably 0.2 to 2%, particularly preferably 0.3 to 2%, and especially preferably 0.4 to 2%. Within the range, even better long-term stability and good usability without stickiness are achieved.

In the cosmetic of the present embodiment, the mass ratio of the component (B) relative to the component (C), (B)/(C), is preferably 1 to 30, more preferably 2 to 28, even more preferably 4 to 25, furthermore preferably 6 to 20, particularly preferably 6 to 16, and especially preferably 6 to 15. Within the range, even better long-term stability and good usability without stickiness are achieved.

In the cosmetic of the present embodiment, the mass ratio of the component (A) relative to the component (C), (A)/(C), is preferably 0.1 to 3.0, more preferably 0.2 to 2.8, even more preferably 0.3 to 2.5, furthermore preferably 0.4 to 2.4, and particularly preferably 0.5 to 2.0. Within the range, even better long-term stability and good usability without stickiness are achieved.

In the cosmetic of the present embodiment, the content of the component (D) is preferably 0.1 to 10% by mass (hereinafter abbreviated as "%"), more preferably 0.3 to 8%, even more preferably 0.5 to 6%, furthermore preferably 0.5 to 4%, and particularly preferably 0.5 to 3%. Within the range, even better long-term stability and good usability without stickiness are achieved.

When the cosmetic of the present embodiment is an oil-in-water emulsion, the cosmetic comprises water. The water is used as a dispersion medium, and examples include, but are not necessarily limited to, purified water, distilled water, ion-exchanged water, tap water, hot spring water, and deep ocean water.

The content of the water is not specifically limited but is preferably 40% or more in the cosmetic.

The oil medium contained in the cosmetic of the present embodiment may be any oil medium that is typically used in cosmetics or similar products, and oily components such as higher alcohols, fatty acids, hydrocarbon oils, ester oils, and silicones may be used. Examples include higher alcohols such as lauryl alcohol, myristyl alcohol, palmityl alcohol, stearyl alcohol, behenyl alcohol, hexadecyl alcohol, oleyl alcohol, isostearyl alcohol, hexyldodecanol, octyldodecanol, cetostearyl alcohol, decyltetradecanol, cholesterol, phytosterol, sitosterol, and lanosterol; fatty acids such as isostearic acid and oleic acid; hydrocarbon oils such as mineral oil, liquid paraffin, light liquid isoparaffin, α-olefin oligomer, plant-derived squalane, squalane, polyisobutylene, polybutene, and hydrogenated polyisobutene; ester oils such as diisobutyl adipate, 2-hexyldecyl adipate, di-2-heptylundecyl adipate, N-alkylglycol monoisostearate, isocetyl isostearate, trimethylolpropane triisostearate, ethylene glycol di-2-ethylhexanoate, cetyl 2-ethylhexanoate, trimethylolpropane tri-2-ethylhexanoate, pentaerythritol tetra-2-ethylhexanoate, cetyl octanoate, oleyl oleate, octyldodecyl oleate, ethyl oleate, decyl oleate, neopentyl glycol dicaprylate, triethyl citrate, 2-ethylhexyl succinate, isocetyl stearate, butyl stearate, diisopropyl sebacate, di-2-ethylhexyl sebacate, cetyl lactate, myristyl lactate, isopropyl palmitate, 2-ethylhexyl palmitate, 2-hexyldecyl palmitate, 2-heptylundecyl palmitate, ethylhexyl salicylate, cholesteryl 12-hydroxystearate, dioctyldodecyl lauroyl glutamate, di(cholesteryl/octyldodecyl) lauroyl glutamate, di(cholesteryl/behenyl/octyldodecyl) lauroyl glutamate, di(octyldodecyl/phytosteryl/behenyl) lauroyl glutamate, (phytosteryl/isostearyl/cetyl/stearyl/behenyl) dimer dilinoleate, di(isostearyl/phytosteryl) dimer dilinoleate, bis-isostearyl dimer dilinoleyl dimer dilinoleate, dimer dilinoleyl diisostearate, dipentaerythrityl (12-hydroxystearate/stearate/rosinate), dipentaerythrityl hexa(hydroxystearate/stearate/rosinate), dipentaerythrityl (12-hydroxystearate/isostearate), cholesteryl isostearate, cholesteryl hydroxystearate, cholesteryl ricinolate, phytosteryl macadamiate, phytosteryl oleate, isopropyl myristate, octyldodecyl myristate, 2-hexyldecyl myristate, myristyl myristate, hexyldecyl dimethyloctanoate, ethyl laurate, hexyl laurate, diisostearyl malate, ethylhexyl hydroxystearate, and glyceryl tri-2-ethylhexanoate; waxes such as beeswax, carnauba wax, candelilla wax, and spermaceti; vegetable oils and fats typified by palm oil, palm kernel oil, olive oil, safflower oil, soybean oil, and cottonseed oil; animal oils such as beef tallow, tallow, bone tallow, hydrogenated tallow, hardened oil, turtle oil, lard, horse fat, mink oil, liver oil, and egg yolk oil; lanolin derivatives such as lanolin, liquid lanolin, reduced lanolin, lanolin alcohol, hard lanolin, lanolin acetate, and isopropyl lanolinate; and silicones such as methylpolysiloxane, methylphenylpolysiloxane, polyoxyalkylene/alkylmethylpolysiloxane/methylpolysiloxane copolymers, alkoxy-modified polysiloxane, alkyl-modified polysiloxane, cross-linked organopolysiloxane, fluorine-modified polysiloxane, amino-modified polysiloxane, glycerol-modified polysiloxane, higher-alkoxy-modified silicone, higher-fatty-acid modified silicone, silicone resin, and silicone resin. These oily components may be used singly or in combination of two or more of them. As the dispersion medium for the dispersion composition in the first embodiment, an ester oil is preferably used.

The cosmetic of the present embodiment may comprise a powder component other than the component (B). The powder contained in the cosmetic is not specifically limited by shape such as a spherical shape, a plate shape, or an acicular shape; particle size such as aerosol, microparticles, or pigment-grade particles; or particle structure such as porous or non-porous. Examples include inorganic powders, glittering powders, organic powders, pigment powders, metal powders, and composite powders. Specific examples include white inorganic pigments such as titanium oxide, zinc oxide, cerium oxide, and barium sulfate; colored inorganic pigments such as iron oxide, carbon black, a titanium/titanium oxide sintered body, chromium oxide, chromium hydroxide, iron blue, and ultramarine; white extender powders such as talc, muscovite, phlogopite, lepidolite, biotite, synthetic mica, sericite, synthetic sericite, silicon carbide, silicon dioxide, aluminum oxide, magnesium oxide, zirconium oxide, antimony oxide, diatomaceous earth, aluminum silicate, magnesium aluminum metasilicate, calcium silicate, barium silicate, magnesium silicate, calcium carbonate, magnesium carbonate, hydroxyapatite, and boron nitride; clay minerals such as kaolin, bentonite, smectite, hectorite, and montmorillonite and organic modified products thereof; glittering powders such as titanium dioxide-coated mica, titanium oxide-coated bismuth oxychloride, iron oxide-mica-titanium, iron blue-treated mica-titanium, carmine-treated mica-titanium, bismuth oxychloride, fish scale foil, polyethylene terephthalate-aluminum-epoxy laminate powder, polyethylene terephthalate-polyolefin laminate film powder, polyethylene terephthalate-polymethyl methacrylate laminate film powder, and titanium oxide-coated glass flakes; copolymer resins such as a polyamide resin, a polyethylene resin, a polyacrylic resin, a polyester resin, a fluorine resin, a cellulose resin, a polystyrene resin, and a styrene-acrylic copolymer resin; powders of organic polymer resins such as a polypropylene resin, a silicone resin, and an urethane resin; organic low-molecular powders such as zinc stearate and N-acyllysine; natural organic powders such as silk powder and cellulose powder; organic pigment powders such as Red No. 201, Red No. 202, Red No. 205, Red No. 226, Red No. 228, Orange No. 203, Orange No. 204, Bue No. 404, and Yellow No. 401; organic pigment powders such as the zirconium, barium, or aluminum lakes of Red No. 3, Red No. 104, Red No. 106, Orange No. 205, Yellow No. 4, Yellow No. 5, Green No. 3, Blue No. 1, and the like; metal powders such as aluminum powder, gold powder, and silver powder; and composite powders such as microparticles of titanium oxide-coated mica-titanium, microparticles of zinc oxide-coated mica-titanium, barium sulfate-coated mica-titanium, titanium oxide-containing silicon dioxide, and zinc oxide-containing silicon dioxide. These powders may be used singly or in combination of two or more of them, and a composite thereof may be used. These powders may be surface-treated with at least one of a fluorine compound, a silicone compound, metallic soap, lecithin, hydrogenated lecithin, collagen, a hydrocarbon, a higher fatty acid, a higher alcohol, an ester, a wax, a waxy solid, and a surfactant.

The cosmetic of the present embodiment may comprise a surfactant. Examples of the surfactant contained in the cosmetic include nonionic surfactants, anionic surfactants, cationic surfactants, and amphoteric surfactants. Examples of the nonionic surfactant include polyoxyethylene fatty acid esters, polyoxyethylene sorbitan fatty acid esters, polyoxyethylene hydrogenated castor oil, hydrogenated castor oil, polyoxyethylene sorbitol tetra-fatty acid esters, glycerol fatty acid esters, sorbitan fatty acid esters, polyglycerol fatty acid esters, sucrose fatty acid esters, alkyl polyglucosides, N-alkyl dimethylamine oxide polyoxyethylene glycerol fatty acid esters, polyoxyethylene cholesteryl ethers, polyoxyethylene phytosteryl ethers, polyoxyethylene alkyl ethers, polyoxyethylene polyoxypropylene alkyl ethers, polyoxyethylene alkyl phenyl ethers, polyether-modified silicone, polyether-alkyl-co-modified silicone, polyether-alkyl-siloxyethyl-co-modified silicone, polyglycerol-modified silicone, polyglycerol-alkyl-co-modified silicone, and polyglycerol-alkyl-siloxyethyl-co-modified silicone. Examples of the anionic surfactant include sodium salts or triethanolamine salts of fatty acids such as lauric acid, myristic acid, palmitic acid, stearic acid, and isostearic acid; N-acyl methyl taurates such as sodium cocoyl methyl taurate, sodium lauroyl methyl taurate, sodium myristoyl methyl taurate, sodium palmitoyl methyl taurate, and sodium stearoyl methyl taurate; and polyoxyethylene alkyl ether phosphate esters such as polyoxyethylene lauryl ether phosphate, polyoxyethylene cetyl ether phosphate, polyoxyethylene oleyl ether phosphate, and polyoxyethylene nonylphenyl ether phosphate. Examples of the cationic surfactant include stearyltrimethylammonium chloride, stearyltrimethylammonium bromide, behenyltrimethylammonium chloride, behenyltrimethylammonium bromide, cetyltrimethylammonium chloride, cetyltrimethylammonium bromide, distearyldimethylammonium chloride, distearyldimethylammonium bromide, dibehenyldimethylammonium chloride, dibehenyldimethylammonium bromide, dicetyldimethylammonium chloride, dicetyldimethylammonium bromide, stearyldimethylbenzylammonium chloride, dilauryldimethylammonium chloride, dipolyoxyethylene (15E.O.) cocoalkylmethylammonium chloride, dipolyoxyethylene (4E.O.) lauryl ether dimethylammonium chloride, stearamidopropyl trimethylammonium chloride, and dicocoylethyl hydroxyethylmethylammonium methyl sulfate. Examples of the amphoteric surfactant include betaines such as cocamidopropyl betaine, octyl dimethylamino acetate betaine, lauramidopropyl betaine, cocoalkyl dimethylamino acetate betaine, myristyl dimethylamino acetate betaine, cetyl dimethylamino acetate betaine, cocamidopropyl dimethylamino acetate betaine, lauramidopropyl dimethylamino acetate betaine, lauryl dihydroxyethylamino acetate betaine, cetyl dihydroxyethylamino acetate betaine, cocoalkyl sulfobetaine, lauryl sulfobetaine, sodium N-cocoacyl-N-carboxymethyl-N-hydroxyethyl ethylenediamine, and disodium N-cocoacyl-N-carboxymethoxyethyl-N-carboxymethyl ethylenediamine; phospholipids such as phosphatidylcholine, phosphatidylethanolamine, phosphatidylserine, phosphatidylglycerol, phosphatidylinositol, sphingophospholipid, soybean lecithin, egg yolk lecithin, and hydrogenated products thereof; and phospholipid complexes such as phospholipid-cholesterol complex and phospholipid-phytosterol complex.

The cosmetic of the present embodiment may comprise an aqueous component. The aqueous component contained in the cosmetic may be any component that is soluble in water, and examples include alcohols such as ethyl alcohol and extraction liquids of plants such as aloe vera, witch hazel, hamamelis, cucumber, lemon, lavender, and rose.

The cosmetic of the present embodiment may comprise a water-soluble polymer. Examples of the water-soluble polymer contained in the cosmetic include natural polymers such as guar gum, sodium chondroitin sulfate, sodium hyaluronate, gum arabic, xanthan gum, sodium alginate, and carrageenan; semisynthetic polymers such as methyl cellulose, hydroxyethyl cellulose, and carboxymethyl cellulose; synthetic polymers such as carboxyvinyl polymers, alkyl-added carboxyvinyl polymers, polyvinyl alcohol, polyvinylpyrrolidone, sodium polyacrylate, (sodium acrylate/sodium acryloyldimethyl taurate) copolymers, (hydroxyethyl acrylate/sodium acryloyldimethyl taurate) copolymers, (acrylates/stearyl methacrylate) copolymers, (acrylates/beheneth-25 methacrylate) copolymers, (PEG-240/decyltetradeceth-20/HDI) copolymers, (ammonium acryloyldimethyltaurate/beheneth-25 methacrylate) crosspolymers, and (ammonium acryloyldimethyltaurate/VP) copolymers; and proteins, mucopolysaccharides, collagen, elastin, and keratin.

The cosmetic of the present embodiment may comprise an additive. Examples of the additive contained in the cosmetic include antioxidants such as α-tocopherol and ascorbic acid, vitamins, antiphlogistics, cosmetic ingredients such as herbal medicines, and antiseptics such as p-hydroxybenzoates and phenoxyethanol.

### (3) Production method of cosmetic

The cosmetic of the present embodiment may be produced by any method: for example, a mixture of the metal oxide dispersion composition comprising the component (A), the component (B), and the component (C) with an optional oil medium is added to a homogeneous mixture of a surfactant and an aqueous component; the whole is emulsified; and then the emulsion is filled into a container or a mold to give the cosmetic.

### (4) Application

The cosmetic of the present embodiment may be in any form, and examples of the form include a liquid form, a semi-solid form, and a solid form. The cosmetic may have any dosage form or any product form. The dosage form may be, for example, a water-in-oil emulsion, an oil-in-water emulsion, a water-dispersion, or a powder, and an oil-in-water emulsion is particularly preferred. Examples of the product form include skin care cosmetics such as a cleansing foam/cream, a cleanser, a massage cream, a face mask, a skin lotion, an emulsion, a cream, a beauty essence, a makeup base, and a sunscreen; finishing cosmetics such as a foundation, a face powder, an eyeshadow, an eyeliner, a mascara, an eyebrow cosmetic, a concealer, a lipstick, and a lip balm; and hair care products such as a hair mist, a shampoo, a hair conditioner, a hair treatment, a hair tonic, a hair cream, a pomade, a hair gel, a liquid styling product, a setting lotion, a hair spray, and a hair dye. Specifically, the effects of the embodiment are more likely to be exerted on cosmetics used for sun protection, such as a sunscreen, a daytime beauty essence, a foundation, and a makeup base.

### 3. Third embodiment

### (1) Formulation of topical skin preparation

A topical skin preparation of the present embodiment comprises the metal oxide dispersion composition in the first embodiment. In the topical skin preparation of the present embodiment, the content of the metal oxide dispersion composition is preferably 5 to 40%, more preferably 10 to 30%, and even more preferably 10 to 20%. In other words, the topical skin preparation of the present embodiment comprises a polyhydroxystearic acid as a component (A), a silicone-treated metal oxide as a component (B), and a glyceryl oleate as a component (C), and the metal oxide in the component (B) is a metal oxide excluding titanium oxide. The components contained in the topical skin preparation will next be described.

### (2) Components

As for the polyhydroxystearic acid as the component (A), the silicone-treated metal oxide as the component (B), the glyceryl oleate as the component (C), and the polyhydric alcohol as the component (D), the above description in the first embodiment also applies to the present embodiment, and a detailed explanation is omitted.

The topical skin preparation of the present embodiment may comprise components used in topical skin preparations, such as water (including purified water, hot spring water, and deep water), an oil medium, a surfactant, a metallic soap, a gelling agent, a powder, a water-soluble polymer, a film-forming agent, a resin, a UV protector, an inclusion compound, an antimicrobial agent, a flavoring agent, a deodorant, a salt, a pH adjuster, an algefacient, an animal or microbial extract, a plant extract, a blood circulation promoter, an astringent, an anti-seborrheic agent, a skin whitening agent, an anti-inflammatory agent, a free radical scavenger, a cell activator, a moisturizer, a chelating agent, a keratolytic agent, an enzyme, a hormone, and a vitamin.

In the topical skin preparation of the present embodiment, the content of the component (A) is preferably 0.05 to 6% by mass (hereinafter abbreviated as "%"), more preferably 0.1 to 4%, even more preferably 0.2 to 3%, furthermore preferably 0.4 to 2.5%, and particularly preferably 0.5 to 2%. Within the range, even better long-term stability and good usability without stickiness are achieved.

In the topical skin preparation of the present embodiment, the mass ratio of the component (B) relative to the total content of the component (A) and the component (C), (B)/{(A) + (C)}, is preferably less than 12, more preferably 10 or less, even more preferably 9 or less, furthermore preferably 8 or less, particularly preferably 7 or less, and especially preferably 6 or less. Within the range, even better usability without stickiness is achieved, and the long-term stability is improved. In the present embodiment, the lower limit of the mass ratio, (B)/{(A) + (C)}, is not specifically limited because the total amount of the component (A) and the component (C) is excess relative to the component (B), and the stability is improved, but is typically preferably 1 or more, more preferably 2 or more, even more preferably 3 or more, furthermore preferably 3.5 or more, and particularly preferably 3.8 or more.

In the topical skin preparation of the present embodiment, the content of the component (B) is preferably 1 to 35% by mass (hereinafter abbreviated as "%"), more preferably 1 to 30%, even more preferably 2 to 28%, furthermore preferably 4 to 20%, and particularly preferably 4 to 15%. Within the range, even better long-term stability and good usability without stickiness are achieved.

In the topical skin preparation of the present embodiment, the content of the component (C) is preferably 0.05 to 6% by mass (hereinafter abbreviated as "%"), more preferably 0.1 to 4%, even more preferably 0.15 to 3%, furthermore preferably 0.2 to 2%, particularly preferably 0.3 to 2%, and especially preferably 0.4 to 2%. Within the range, even better long-term stability and good usability without stickiness are achieved.

In the topical skin preparation of the present embodiment, the mass ratio of the component (B) relative to the component (C), (B)/(C), is preferably 1 to 30, more preferably 2 to 28, even more preferably 4 to 25, furthermore preferably 6 to 20, particularly preferably 6 to 16, and especially preferably 6 to 15. Within the range, even better long-term stability and good usability without stickiness are achieved.

In the topical skin preparation of the present embodiment, the mass ratio of the component (A) relative to the component (C), (A)/(C), is preferably 0.1 to 3.0, more preferably 0.2 to 2.8, even more preferably 0.3 to 2.5, furthermore preferably 0.4 to 2.4, and particularly preferably 0.5 to 2.0. Within the range, even better long-term stability and good usability without stickiness are achieved.

In the topical skin preparation of the present embodiment, the content of the component (D) is preferably 0.1 to 10% by mass (hereinafter abbreviated as "%"), more preferably 0.3 to 8%, even more preferably 0.5 to 6%, furthermore preferably 0.5 to 4%, and particularly preferably 0.5 to 3%. Within the range, even better long-term stability and good usability without stickiness are achieved.

### (3) Production method of topical skin preparation

The topical skin preparation of the present embodiment may be produced by any method and is produced by a conventional method. In an example method, the components (A) to (C) are mixed; the above optional components are added as needed; and the whole is mixed.

### (4) Application

The topical skin preparation of the present embodiment may be in various forms such as a liquid form, a gel form, a cream form, a solid form, and a mousse form. Alternatively, the topical skin preparation may be contained in a container configured to spray the preparation as a mist. The dosage form of the present embodiment may be any form, and examples include a solubilized form, an oil-in-water emulsion, a water-in-oil emulsion, an oil form, a water-in-oil-in-water form, an oil-in-water-in-oil form, and a multilayer form. An oil-in-water emulsion is particularly preferred.

The topical skin preparation of the present embodiment may be used in any application and may be used as a topical skin preparation excellent in antiaging or other effects.

Examples of the form of the topical skin preparation of the present embodiment include, but are not necessarily limited to, skin care cosmetics such as an emulsion, a cream, a skin lotion, a beauty essence, a sunscreen, and a face mask; makeup cosmetics such as a foundation, a cheek rouge, a lipstick, an eye color, a mascara, an eyeliner, and a nail polish; hair care cosmetics such as a hair growth agent, a hair tonic, a shampoo, a hair conditioner, and a hair wax; cleansers such as a facial cleanser; and a topical gel, a cream, an ointment, a liquid, a liniment, a gel patch, and an aerosol. Specifically, the effects of the embodiment are more likely to be exerted on topical skin preparations used for sun protection, such as a sunscreen, a foundation, and a daytime beauty essence.

### 4. Fourth embodiment

### (1) Formulation of cosmetic

A cosmetic of the present embodiment comprises a polyhydroxystearic acid as a component (A), a silicone-treated metal oxide as a component (B), and a glyceryl oleate as a component (C), and the metal oxide in the component (B) is a metal oxide excluding titanium oxide.

### (2) Components

As for the polyhydroxystearic acid as the component (A), the silicone-treated metal oxide as the component (B), the glyceryl oleate as the component (C), and the polyhydric alcohol as the component (D), the above description in the second embodiment also applies to the present embodiment, and a detailed explanation is omitted.

The cosmetic of the present embodiment may appropriately comprise, within the range not impairing the effects of the present embodiment, in addition to the above components, components typically contained in cosmetics, including water, an oil medium, powders such as an inorganic pigment, an organic pigment, and an extender pigment, a surfactant, aqueous components such as a lower alcohol, a water-soluble polymer, and a moisturizer, a sugar, an ultraviolet absorber, an antioxidant, an anti-fading agent, an antiseptic, a pharmaceutical ingredient, a stabilizer, a pigment, and a flavoring agent, as needed, for imparting various effects. As for the components other than the above components, including water, an oil medium, powders such as an inorganic pigment, an organic pigment, and an extender pigment, a surfactant, aqueous components such as a lower alcohol, a water-soluble polymer, and a moisturizer, a sugar, an ultraviolet absorber, an antioxidant, an anti-fading agent, an antiseptic, a pharmaceutical ingredient, a stabilizer, a pigment, and a flavoring agent, the above description in the second embodiment also applies to the present embodiment, and a detailed explanation is omitted.

As for the contents of the components and the mass ratio of the components, the above description in the second embodiment also applies to the present embodiment, and a detailed explanation is omitted.

### (3) Production method of cosmetic

The cosmetic of the present embodiment may be produced by any method: for example, a mixture of the component (A), the component (B), the component (C), and an optional oil medium is added to a homogeneous mixture of a surfactant and an aqueous component; the whole is emulsified; and then the emulsion is filled into a container or a mold to give the cosmetic.

### (4) Application

As for the application of the cosmetic of the present embodiment, the above description in the second embodiment also applies to the present embodiment, and a detailed explanation is omitted.

### 5. Fifth embodiment

### (1) Formulation of topical skin preparation

A topical skin preparation of the present embodiment comprises a polyhydroxystearic acid as a component (A), a silicone-treated metal oxide as a component (B), and a glyceryl oleate as a component (C), and the metal oxide in the component (B) is a metal oxide excluding titanium oxide.

### (2) Components

As for the polyhydroxystearic acid as the component (A), the silicone-treated metal oxide as the component (B), the glyceryl oleate as the component (C), and the polyhydric alcohol as the component (D), the above description in the third embodiment also applies to the present embodiment, and a detailed explanation is omitted.

The topical skin preparation of the present embodiment may comprise components used in topical skin preparations, such as water (including purified water, hot spring water, and deep water), an oil medium, a surfactant, a metallic soap, a gelling agent, a powder, a water-soluble polymer, a film-forming agent, a resin, a UV protector, an inclusion compound, an antimicrobial agent, a flavoring agent, a deodorant, a salt, a pH adjuster, an algefacient, an animal or microbial extract, a plant extract, a blood circulation promoter, an astringent, an anti-seborrheic agent, a skin whitening agent, an anti-inflammatory agent, a free radical scavenger, a cell activator, a moisturizer, a chelating agent, a keratolytic agent, an enzyme, a hormone, and a vitamin. As for the components such as water (including purified water, hot spring water, and deep water), an oil medium, a surfactant, a metallic soap, a gelling agent, a powder, a water-soluble polymer, a film-forming agent, a resin, a UV protector, an inclusion compound, an antimicrobial agent, a flavoring agent, a deodorant, a salt, a pH adjuster, an algefacient, an animal or microbial extract, a plant extract, a blood circulation promoter, an astringent, an anti-seborrheic agent, a skin whitening agent, an anti-inflammatory agent, a free radical scavenger, a cell activator, a moisturizer, a chelating agent, a keratolytic agent, an enzyme, a hormone, and a vitamin, the above description in the third embodiment also applies to the present embodiment, and a detailed explanation is omitted.

As for the contents of the components and the mass ratio of the components, the above description in the third embodiment also applies to the present embodiment, and a detailed explanation is omitted.

### (3) Production method of topical skin preparation

The topical skin preparation of the present embodiment may be produced by any method and is produced by a conventional method. In an example method, the components (A) to (C) are mixed; the above optional components are added as needed; and the whole is mixed.

### (4) Application

As for the application of the topical skin preparation of the present embodiment, the above description in the third embodiment also applies to the present embodiment, and a detailed explanation is omitted.

The present invention may include, in addition to the above embodiments, the following aspects.
[1] A metal oxide dispersion composition comprising components (A) to (C):
   (A) a polyhydroxystearic acid,
   (B) a silicone-treated metal oxide, and
   (C) a glyceryl oleate,
   in which the metal oxide in the component (B) is a metal oxide excluding titanium oxide.
[2] The metal oxide dispersion composition according to the aspect [1], in which the mass ratio of the component (B) relative to the total content of the component (A) and the component (C), (B)/{(A) + (C)}, is less than 12.
[3] The metal oxide dispersion composition according to the aspect [1] or [2], in which the component (B) is a dimethylpolysiloxane-treated metal oxide.
[4] The metal oxide dispersion composition according to any one of the aspects [1] to [3], in which the content of the component (B) is 20 to 80% by mass.
[5] The metal oxide dispersion composition according to any one of the aspects [1] to [4], in which the mass ratio of the component (B) relative to the component (C), (B)/(C), is 1 to 30.
[6] The metal oxide dispersion composition according to any one of the aspects [1] to [5], in which the mass ratio of the component (A) relative to the component (C), (A)/(C), is 0.1 to 3.0.
[7] The metal oxide dispersion composition according to any one of the aspects [1] to [6], in which the metal oxide in the component (B) is zinc oxide.
[8] The metal oxide dispersion composition according to any one of the aspects [1] to [7], further comprising a component (D), a polyhydric alcohol.
[9] A cosmetic or a topical skin preparation comprising the metal oxide dispersion composition according to any one of the aspects [1] to [8].
[10] The cosmetic or the topical skin preparation according to the aspect [9], in which the cosmetic or the topical skin preparation is an oil-in-water emulsion.
[11] The cosmetic or the topical skin preparation according to the aspect [9] or [10], in which the cosmetic or the topical skin preparation is used for sun protection.
[12] A cosmetic or a topical skin preparation comprising components (A) to (C):
   (A) a polyhydroxystearic acid,
   (B) a silicone-treated metal oxide, and
   (C) a glyceryl oleate,
   in which the metal oxide in the component (B) is a metal oxide excluding titanium oxide.
[13] The cosmetic or the topical skin preparation according to the aspect [12], in which the mass ratio of the component (B) relative to the total content of the component (A) and the component (C), (B)/{(A) + (C)}, is less than 12.
[14] The cosmetic or the topical skin preparation according to the aspect [12] or [13], in which the component (B) is a dimethylpolysiloxane-treated metal oxide.
[15] The cosmetic or the topical skin preparation according to any one of the aspects [12] to [14], in which the content of the component (B) is 1 to 35% by mass.
[16] The cosmetic or the topical skin preparation according to any one of the aspects [12] to [15], in which the mass ratio of the component (B) relative to the component (C), (B)/(C), is 1 to 30.
[17] The cosmetic or the topical skin preparation according to any one of the aspects [12] to [16], in which the mass ratio of the component (A) relative to the component (C), (A)/(C), is 0.1 to 3.0.
[18] The cosmetic or the topical skin preparation according to any one of the aspects [12] to [17], in which the metal oxide in the component (B) is zinc oxide.
[19] The cosmetic or the topical skin preparation according to any one of the aspects [12] to [18], further comprising a component (D), a polyhydric alcohol.
[20] The cosmetic or the topical skin preparation according to the aspect [19], in which the component (D) is glycerol.
[21] The cosmetic or the topical skin preparation according to any one of the aspects [12] to [20], in which the cosmetic or the topical skin preparation is an oil-in-water emulsion.
[22] The cosmetic or the topical skin preparation according to any one of the aspects [12] to [21], in which the cosmetic or the topical skin preparation is used for sun protection.

### 6. Examples

The present invention will next be described in more detail with reference to examples, but the present invention is not limited to these examples. Unless otherwise specified, the content is expressed as % by mass relative to a system containing the component.

### 6-1. Production of metal oxide dispersion composition

### [Examples 1 to 7, Comparative Examples 1 to 5]

Metal oxide dispersion compositions having the following formulations shown in Table 1 were prepared by the production method described below. The "dispersibility" of each sample was evaluated by the evaluation method described below on the basis of the criteria below, and the results are also shown in Table 1.

**[Table 1]**

| % by mass | | | | | | | | | | | | | | |
|---|---|---|---|---|---|---|---|---|---|---|---|---|---|---|
| No. | component | name | Example | | | | | | | Comparative Example | | | | |
| | | | 1 | 2 | 3 | 4 | 5 | 6 | 7 | 1 | 2 | 3 | 4 | 5 |
| 1 | (A) | polyhydroxystearic acid (note 1) | 6 | 9 | 3 | 6 | 6 | 4 | 6 | - | - | 6 | - | 6 |
| 2 | (A), comparative | PEG-30 dipolyhydroxystearate (note 2) | - | - | - | - | - | - | - | 6- | | - | - | - |
| 3 | | polyglyceryl-5 polyricinoleate (note 3) | - | - | - | - | - | - | - | - | 6 | - | - | - |
| 4 | (B) | dimethylpolysiloxane-treated zinc oxide (note 4) | 60 | 60 | 60 | 60 | 60 | 40 | 80 | 60 | 60 | 60 | 60 | |
| 5 | (B), comparative | dimethylpolysiloxane-treated titanium oxide (note 10) | | - | - | - | - | - | - | - | - | - | - | 60 |
| 6 | (C) | glyceryl oleate | 5 | 5 | 5 | 10 | 2.5 | 3.5 | 5 | 5 | 5 | - | 5 | 5 |
| 7 | | cetyl 2-ethylhexanoate | remain | remain | remain | remain | remain | remain | remain | remain | remain | remain | remain | remain |
| | | mass ratio (A)/(C) | 1.2 | 1.8 | 0.6 | 0.6 | 2.4 | 1.1 | 1.2 | - | - | - | - | 1.2 |
| | | mass ratio (B)/(C) | 12 | 12 | 12 | 6.0 | 24 | 11 | 16 | 12 | 12 | - | 12 | - |
| | | mass ratio (B)/{(A)+(C)} | 5.5 | 4.3 | 7.5 | 3.8 | 7.1 | 5.3 | 7.3 | 12 | 12 | 10 | 12 | - |
| Evaluation item | | dispersibility | A | A | B | A | B | A | B | C | C | C | C | C |

| | | | | | | | | | | | | | | |
|---|---|---|---|---|---|---|---|---|---|---|---|---|---|---|
| (note 1) SALACOS HS-6C (manufactured by The Nisshin OilliO Group) (note 2) CITHROL DPHS (manufactured by Croda Japan KK.) (note 3) Sunsoft No. 818R-C (manufactured by Taiyo Kagaku Co., Ltd.) (note 4) The used zinc oxide was MZ-500 (manufactured by Tayca Corporation; average particle size: 25 nm), and the treatment amount of dimethylpolysiloxane was 5% relative to the mass of zinc oxide. (note 10) MTY-110M3S (manufactured by Tayca Corporation) | | | | | | | | | | | | | | |

### (Production method)

The components (1) to (7) were homogeneously mixed, and the mixture was dispersed with a roll mill to give a metal oxide dispersion composition.

### <Evaluation method 1: dispersibility>

A metal oxide dispersion composition containing a well-dispersed metal oxide has a low viscosity. For the dispersibility in the present invention, a produced metal oxide dispersion composition was stored in a constant temperature chamber at 30°C for a day; then the viscosity of the metal oxide dispersion composition was determined with a Brookfield viscometer; and the dispersibility was evaluated on the basis of the following three-level grading system.

### <Three-level grading system for dispersibility of metal oxide dispersion composition>

| [Grade]: | [Criteria] |
|---|---|
| A (excellent): | The viscosity is less than 10,000 mPa·s. |
| B (good): | The viscosity is not less than 10,000 mPa·s and less than 20,000 mPa·s. |
| C (poor): | The viscosity is not less than 20,000 mPa·s. |

As shown in Table 1, the metal oxide dispersion compositions in Examples 1 to 7 each containing dimethylpolysiloxane-treated zinc oxide as the silicone-treated metal oxide, polyhydroxystearic acid, and glyceryl oleate had excellent "dispersibility". In contrast, the metal oxide dispersion composition in Comparative Example 1 containing PEG-30 dipolyhydroxystearate in place of polyhydroxystearic acid, the metal oxide dispersion composition in Comparative Example 2 containing polyglyceryl-5 polyricinoleate in place of polyhydroxystearic acid, the metal oxide dispersion composition in Comparative Example 3 prepared in a similar manner to that in Example 1 except that glyceryl oleate was not contained, the metal oxide dispersion composition in Comparative Example 4 prepared in a similar manner to that in Example 1 except that polyhydroxystearic acid was not contained, and the metal oxide dispersion composition in Comparative Example 5 containing a silicone-treated titanium oxide (MTY-110M3S, manufactured by Tayca Corporation) in place of dimethylpolysiloxane-treated zinc oxide each had poor "dispersibility" as compared to Example 1.

### 4-2. Production of cosmetic

### [Examples 8 to 14, Comparative Examples 6 to 11]

Oil-in-water emulsion cosmetics (sunscreens) having the formulations shown in Table 2 were prepared by the production method described below. "Long-term stability (at 40°C for a month)" and "no stickiness" of each cosmetic were evaluated by the evaluation method described below on the basis of the criteria below, and the results are also shown in Table 2. A photograph of appearances of the oil-in-water emulsion cosmetics in Example 8 and Comparative Example 6 after the long-term stability test (at 40°C for a month) is shown in Fig. 1. In Comparative Example 6, the metal oxide dispersion composition in Comparative Example 1 was used in place of the metal oxide dispersion composition in Example 1 to prepare an oil-in-water emulsion cosmetic by the production method described below; in Comparative Example 7, the metal oxide dispersion composition in Comparative Example 2 was used in place of the metal oxide dispersion composition in Example 1 to prepare an oil-in-water emulsion cosmetic by the production method described below; in Comparative Example 8, the metal oxide dispersion composition in Comparative Example 3 was used in place of the metal oxide dispersion composition in Example 1 to prepare an oil-in-water emulsion cosmetic by the production method described below; in Comparative Example 9, the metal oxide dispersion composition in Comparative Example 4 was used in place of the metal oxide dispersion composition in Example 1 to prepare an oil-in-water emulsion cosmetic by the production method described below; in Comparative Example 10, the metal oxide dispersion composition in Comparative Example 5 was used in place of the metal oxide dispersion composition in Example 1 to prepare an oil-in-water emulsion cosmetic by the production method described below; and in Comparative Example 11, a metal oxide dispersion composition (Reference Example 1), which was prepared in a similar manner to that in Example 1 except that glyceryl stearate was used in place of glyceryl oleate, was used to prepare an oil-in-water emulsion cosmetic by the production method described below.

**[Table 2]**

| | | | | | | | | | | % by mass | | | | | |
|---|---|---|---|---|---|---|---|---|---|---|---|---|---|---|---|
| No. | component | name | Example | | | | | | | Comparative Example | | | | | |
| | | | 8 | 9 | 10 | 11 | 12 | 13 | 14 | 6 | 7 | 8 | 9 | 10 | 11 |
| 1 | metal oxide dispersion composition | metal oxide dispersion composition in Example 1 | 17 | - | - | - | - | - | - | - | - | - | - | - | - |
| 2 | | metal oxide dispersion composition in Example 2 | - | 17 | - | - | - | - | - | - | - | - | - | - | - |
| 3 | | metal oxide dispersion composition in Example 3 | - | - | 17 | - | - | - | - | - | - | - | - | - | - |
| 4 | | metal oxide dispersion composition in Example 4 | - | - | - | 17 | - | - | - | - | - | - | - | - | - |
| 5 | | metal oxide dispersion composition in Example 5 | - | - | - | - | 17 | - | - | - | - | - | - | - | - |
| 6 | | metal oxide dispersion composition in Example 6 | - | - | - | - | - | 17 | - | - | - | - | - | - | - |
| 7 | | metal oxide dispersion composition in Example 7 | - | - | - | - | - | - | 17 | - | - | - | - | - | - |
| 8 | comparative metal oxide dispersion composition | metal oxide dispersion composition in Comparative Example 1 | - | - | - | - | - | - | - | 17 | - | - | - | - | - |
| 9 | | metal oxide dispersion composition in Comparative Example 2 | - | - | - | - | - | - | - | - | 17 | - | - | - | - |
| 10 | | metal oxide dispersion composition in Comparative Example 3 | - | - | - | - | - | - | - | - | - | 17 | - | - | - |
| 11 | | metal oxide dispersion composition in Comparative Example 4 | - | - | - | - | - | - | - | - | - | - | 17 | - | - |
| 12 | | metal oxide dispersion composition in Comparative Example 5 | - | - | - | - | - | - | - | - | - | - | - | 17 | - |
| 13 | | metal oxide dispersion composition in Reference Example 1 | - | - | - | - | - | - | - | - | - | - | - | - | 17 |
| 14 | | bis-ethylhexyloxyphenol methoxyphenyl triazine (note 5) | 2 | 2 | 2 | 2 | 2 | 2 | 2 | 2 | 2 | 2 | 2 | 2 | 2 |
| 15 | | ethylhexyl methoxycinnamate | 7 | 7 | 7 | 7 | 7 | 7 | 7 | 7 | 7 | 7 | 7 | 7 | 7 |
| 16 | | diethylamino hydroxybenzoyl hexyl benzoate (note 6) | 0.5 | 0.5 | 0.5 | 0.5 | 0.5 | 0.5 | 0.5 | 0.5 | 0.5 | 0.5 | 0.5 | 0.5 | 0.5 |
| 17 | | (sodium acrylate/sodium acryloyldimethyl taurate) copolymer (note 7) | 0.3 | 0.3 | 0.3 | 0.3 | 0.3 | 0.3 | 0.3 | 0.3 | 0.3 | 0.3 | 0.3 | 0.3 | 0.3 |
| 18 | | PEG-80 hydrogenated castor oil | 1 | 1 | 1 | 1 | 1 | 1 | 1 | 1 | 1 | 1 | 1 | 1 | 1 |
| 19 | | phenoxyethanol | 0.2 | 0.2 | 0.2 | 0.2 | 0.2 | 0.2 | 0.2 | 0.2 | 0.2 | 0.2 | 0.2 | 0.2 | 0.2 |
| 20 | | ethanol | 4 | 4 | 4 | 4 | 4 | 4 | 4 | 4 | 4 | 4 | 4 | 4 | 4 |
| 21 | | silica (note 8) | 1 | 1 | 1 | 1 | 1 | 1 | 1 | 1 | 1 | 1 | 1 | 1 | 1 |
| 22 | | purified water | remain | remain | remain | remain | remain | remain | remain | remain | remain | remain | remain | remain | remain |
| | | content of component (A) relative to total amount of cosmetic | 1.0 | 1.5 | 0.5 | 1.0 | 1.0 | 0.7 | 1.0 | - | - | 1.0 | - | 1.0 | 1.0 |
| | | content of component (B) relative to total amount of cosmetic | 10 | 10 | 10 | 10 | 10 | 7 | 14 | 10 | 10 | 10 | 10 | - | 10 |
| | | content of component (C) relative to total amount of cosmetic | 0.9 | 0.9 | 0.9 | 1.7 | 0.4 | 0.6 | 0.9 | 0.9 | 0.9 | - | 0.9 | 0.9 | - |
| | | mass ratio (A)/(C) | 1.2 | 1.8 | 0.6 | 0.6 | 2.4 | 1.1 | 1.2 | - | - | - | - | 1.2 | - |
| | | mass ratio (B)/(C) | 12 | 12 | 12 | 6 | 24 | 11 | 16 | 12 | 12 | - | 12 | - | - |
| | | mass ratio (B)/{(A)+(C)} | 5.5 | 4.3 | 7.5 | 3.8 | 7.1 | 5.3 | 7.3 | 12 | 12 | 10 | 12 | - | 10 |
| Evaluation item | | long-term stability (at 40°C for a month) | A | A | B | A | B | A | B | C | C | C | C | C | B |
| | | no stickiness | A | B | A | B | A | A | B | D | D | D | D | D | D |

| | | | | | | | | | | | | | | | |
|---|---|---|---|---|---|---|---|---|---|---|---|---|---|---|---|
| (note 5) TINOSORB S (manufactured by BASF) (note 6) Uvinul A PLUS GRANULAR (manufactured by BASF) (note 7) SIMULGEL EG QD (manufactured by SEPPIC) (purity: 37.5%) (note 8) Godd Ball D11-796C (manufactured by SUZUKIYUSHI | | | | | | | | | | | | | | | |

### INDUSTRIAL CORPORATION)

### (Production method)

A: The components (14) to (16) were mixed, and the components (1) to (13) were further added. The whole was mixed and dispersed to give a mixture (A).
B: The components (17) to (22) were homogeneously mixed, and the mixture (A) was further added. The whole was emulsified and degassed to give an oil-in-water emulsion cosmetic.

### <Evaluation method 2: long-term stability (at 40°C for a month)>

If a metal oxide dispersion composition or an oil-in-water emulsion cosmetic has a good stability, the change in viscosity over time described below is small. If a metal oxide dispersion composition or an oil-in-water emulsion cosmetic has a poor stability, the change in viscosity over time is large (gelation occurs). For the long-term stability (at 40°C for a month) in the present invention, a produced cosmetic was stored in a constant temperature chamber at 30°C for a day; and the viscosity was determined with a Brookfield viscometer. Separately, a produced cosmetic was stored in a constant temperature chamber at 40°C for a month and then was stored in a constant temperature chamber at 30°C for a day; and the viscosity was determined with a Brookfield viscometer. The change in viscosity was evaluated on the basis of the following three-level grading system.

### <Three-level grading system for long-term stability>

| [Grade]: | [Criteria] |
|---|---|
| A (excellent): | The change in viscosity is not less than 0 mPa ·s and less than ± 5,000 mPa·s. |
| B (good): | The change in viscosity is not less than ± 5,000 mPa ·s and less than ± 15,000mPa ·s. |
| C (poor): | The change in viscosity is not less than ± 15,000 mPa ·s. |

### <Evaluation method 3: no stickiness>

For no stickiness, the following sensory evaluation test was performed. Among 20s to 40s females having received training in sensory evaluation, a specialist panel of 10 females capable of evaluating samples on the basis of certain criteria were selected. To the forearms of the specialist panel, 2 g of each sample was applied, and no stickiness upon application was rated on the basis of the following 5-point absolute evaluation. For each sample, the average score was calculated from the total scores of all the panel members, and the sample was evaluated on the basis of the following four-level grading system.

### <Absolute evaluation system>

| [Score]: | [Criteria] |
|---|---|
| 5 points: | not sticky |
| 4 points: | barely sticky |
| 3 points: | slightly sticky |
| 2 points: | sticky |
| 1 point: | very sticky |

### <Four-level grading system>

| [Grade]: | [Average score] |
|---|---|
| A (excellent): | more than 4.5 points |
| B (good): | more than 3.5 points and not more than 4.5 points |
| C (fair): | more than 1.5 points and not more than 3.5 points |
| D (poor): | not more than 1.5 points |

As shown in Table 2, the oil-in-water emulsion cosmetics in Examples 8 to 14 each containing the metal oxide dispersion composition in Examples 1 to 7, which contains dimethylpolysiloxane-treated zinc oxide as the silicone-treated metal oxide, polyhydroxystearic acid, and glyceryl oleate, were excellent in both "long-term stability" and "no stickiness". In contrast, the oil-in-water emulsion cosmetic in Comparative Example 6 containing the metal oxide dispersion composition in Comparative Example 1, the oil-in-water emulsion cosmetic in Comparative Example 7 containing the metal oxide dispersion composition in Comparative Example 2, the oil-in-water emulsion cosmetic in Comparative Example 8 containing the metal oxide dispersion composition in Comparative Example 3, the oil-in-water emulsion cosmetic in Comparative Example 9 containing the metal oxide dispersion composition in Comparative Example 4, and the oil-in-water emulsion cosmetic in Comparative Example 10 containing the metal oxide dispersion composition in Comparative Example 5, in place of the metal oxide dispersion composition in Examples 1 to 7, were inferior in both "long-term stability" and "no stickiness" to the oil-in-water emulsion cosmetics in Examples 8 to 14. As shown in Fig. 1 (left), no gelation was observed in the oil-in-water emulsion cosmetic in Example 8 even after storage in a constant temperature chamber at 40°C for a month, and this reveals excellent long-term stability.

In contrast, as shown in Fig. 1 (right), gelation was observed in Comparative Example 6 in which the metal oxide dispersion composition in Comparative Example 1 was used in place of the metal oxide dispersion composition in Example 1, and this reveals insufficient long-term stability. The oil-in-water emulsion cosmetic in Comparative Example 11 containing the metal oxide dispersion composition (Reference Example 1), which was prepared by using glyceryl stearate in place of glyceryl oleate in Example 1, gave very sticky feeling upon application and had insufficient usability.

### [Example 15: daytime beauty essence (oil-in-water emulsion)]

An oil-in-water emulsion cosmetic (daytime beauty essence) having the following formulation was prepared by the production method described below.

| (Component) | (% by mass) |
|---|---|
| 1. Microparticles of dimethylpolysiloxane (10%-treated) zinc oxide (10 nm) (component (B)) | 5 |
| 2. Polyhydroxystearic acid (note 1) (component (A)) | 1 |
| 3. Glyceryl oleate (component (C)) | 1 |
| 4. Cetyl 2-ethylhexanoate | 3 |
| 5. Ethylhexyl methoxycinnamate | 7 |
| 6. Diethylamino hydroxybenzoyl hexyl benzoate (note 6) | 2 |
| 7. 2,4-Bis{[4-(2-ethylhexyloxy)-2-hydroxyl-phenyl}-6-(4-meth oxyphenyl)-1,3,5-triazine (note 5) | 1 |
| 8. Propylene glycol dicaprylate/dicaprate | 5 |
| 9. Niacinamide | 5 |
| 10. Tranexamic acid | 2 |
| 11. (Sodium acrylate/sodium acryloyldimethyl taurate) copol ymer (note 7) | 0.5 |
| 12. Xanthan gum | 0.1 |
| 13. Sodium hyaluronate (note 9) | 0.05 |
| 14. Glycerol (component (D)) | 2 |
| 15. Dipropylene glycol (component (D)) | 2 |
| 16. 1,3-Butylene glycol (component (D)) | 2 |
| 17. Vegetable squalane | 0.5 |
| 18. PEG-10 hydrogenated castor oil | 0.1 |
| 19. PEG-80 hydrogenated castor oil | 1.5 |
| 20. PEG-40 hydrogenated castor oil | 0.3 |
| 21. Ethanol | 5 |
| 22. Purified water | remain |

| | |
|---|---|
| (note 9) Hyaluronic Acid FCH-SU (Kikkoman Biochemifa Company) | |

### (Production method)

A: The components (1) to (4) were mixed and dispersed with a roll mill to give a metal oxide dispersion composition (A).
B: The components (5) to (8) were heated at 75°C and mixed, and the composition (A) was further added. The whole was mixed to give a mixture (B).
C: The components (9) to (22) were homogeneously mixed, and the resulting mixture was added to the mixture (B). The whole was emulsified to give a daytime beauty essence (oil-in-water emulsion).

The daytime beauty essence (oil-in-water emulsion) in Example 15 had excellent long-term stability and no sticky feeling upon use.

### [Example 16: daytime beauty essence (oil-in-water emulsion)]

An oil-in-water emulsion cosmetic (daytime beauty essence) having the following formulation was prepared by the production method described below.

| (Component) | (% by mass) |
|---|---|
| 1. Microparticles of stearic acid/aluminum hydroxide-treated (10 nm) titanium oxide | 10 |
| 2. Microparticles of dimethylpolysiloxane (10%-treated) zinc (component (B)) oxide (25 nm) | 10 |
| 3. Polyhydroxystearic acid (note 1) (component (A)) | 1 |
| 4. Glyceryl oleate (component (C)) | 1 |
| 5. Glycerol (component (D)) | 0.2 |
| 6. Cetyl 2-ethylhexanoate | 5 |
| 7. Propylene glycol dicaprate | 3 |
| 8. Stearyl glycyrrhetinate | 0.1 |
| 9. (Sodium acrylate/sodium acryloyldimethyl taurate) copoly mer (note 7) | 0.5 |
| 10. Xanthan gum | 0.1 |
| 11. Sodium hyaluronate (note 9) | 0.05 |
| 12. Glycerol (component (D)) | 2 |
| 13. Dipropylene glycol (component (D)) | 2 |
| 14. 1,3-Butylene glycol (component (D)) | 2 |
| 15. PEG-80 hydrogenated castor oil | 2 |
| 16. Ethanol | 5 |
| 17. Purified water | remain |

### (Production method)

A: The components (1) to (6) were mixed and dispersed with a roll mill to give a metal oxide dispersion composition (A).
B: The components (7) to (8) were mixed, and the composition (A) was further added. The whole was mixed to give a mixture (B).
C: The components (9) to (17) were homogeneously mixed, and the resulting mixture was added to the mixture (B). The whole was emulsified to give a daytime beauty essence (oil-in-water emulsion).

The daytime beauty essence (oil-in-water emulsion) in Example 16 had excellent long-term stability and no sticky feeling upon use.

### [Example 17: daytime beauty essence (oil-in-water emulsion)]

An oil-in-water emulsion cosmetic (daytime beauty essence) having the following formulation was prepared by the production method described below.

| (Component) | (% by mass) |
|---|---|
| 1. Microparticles of dimethylpolysiloxane (10%-treated) zinc (component (B)) oxide (10 nm) | 5 |
| 2. Polyhydroxystearic acid (note 1) (component (A)) | 1 |
| 3. Glyceryl oleate (component (C)) | 1 |
| 4. Cetyl 2-ethylhexanoate | 3 |
| 5. Ethylhexyl methoxycinnamate | 7 |
| 6. Diethylamino hydroxybenzoyl hexyl benzoate (note 6) | 2 |
| 7. 2,4-Bis{[4-(2-ethylhexyloxy)-2-hydroxy]-phenyl}-6-(4-meth 1,3,5-triazine (note 5) oxyphenyl)- | |
| 8. Propylene glycol dicaprylate/dicaprate | 5 |
| 9. Niacinamide | 5 |
| 10. Tranexamic acid | 2 |
| 11. (Sodium acrylate/sodium acryloyldimethyl taurate) copolymer(note 7) | 0.5 |
| 12. Xanthan gum | 0.1 |
| 13. Sodium hyaluronate (note 9) | 0.05 |
| 14. Glycerol (component (D)) | 0.5 |
| 15. Dipropylene glycol (component (D)) | 1 |
| 16. 1,3-Butylene glycol (component (D)) | 1.5 |
| 17. Vegetable squalane | 0.5 |
| 18. Sodium stearate | 1.5 |
| 19. Polyglyceryl-10 stearate | 0.5 |
| 20. Sodium methyl stearoyl taurate | 0.3 |
| 21. Ethanol | 5 |
| 22. Purified water | remain |

### (Production method)

A: The components (1) to (4) were mixed and dispersed with a roll mill to give a metal oxide dispersion composition (A).
B: The components (5) to (8) were mixed, and the composition (A) was further added. The whole was mixed to give a mixture (B).
C: The components (9) to (22) were homogeneously mixed, and the resulting mixture was added to the mixture (B). The whole was emulsified to give a daytime beauty essence (oil-in-water emulsion).

The daytime beauty essence (oil-in-water emulsion) in Example 17 had excellent long-term stability and no sticky feeling upon use.

### [Example 18: daytime beauty essence (oil-in-water emulsion)]

An oil-in-water emulsion cosmetic (daytime beauty essence) having the following formulation was prepared by the production method described below.

| (Component) | (% by mass) |
|---|---|
| 1. Microparticles of stearic acid/aluminum hydroxide-treate (10 nm) d titanium oxide | 10 |
| 2. Microparticles of dimethylpolysiloxane (10%-treated) zincoxide (25 nm) (component (B)) | 10 |
| 3. Polyhydroxystearic acid (note 1) (component (A)) | 1 |
| 4. Glyceryl oleate (component (C)) | 1 |
| 5. Glycerol (component (D)) | 0.4 |
| 6. Cetyl 2-ethylhexanoate | 5 |
| 7. Propylene glycol dicaprate | 3 |
| 8. Stearyl glycyrrhetinate | 0.1 |
| 9. (Sodium acrylate/sodium acryloyldimethyl taurate) copolymer (note 7) | 0.5 |
| 10. Xanthan gum | 0.1 |
| 11. Sodium hyaluronate (note 9) | 0.05 |
| 12. Glycerol (component (D)) | 1 |
| 13. Dipropylene glycol (component (D)) | 1 |
| 14. 1,3-Butylene glycol (component (D)) | 1 |
| 15. PEG-80 hydrogenated castor oil | 2 |
| 16. Ethanol | 5 |
| 17. Purified water | remain |

### (Production method)

A: The components (1) to (6) were mixed and dispersed with a roll mill to give a metal oxide dispersion composition (A).
B: The components (7) to (8) were mixed, and the composition (A) was further added. The whole was mixed to give a mixture (B).
C: The components (9) to (17) were homogeneously mixed, and the resulting mixture was added to the mixture (B). The whole was emulsified to give a daytime beauty essence (oil-in-water emulsion).

The daytime beauty essence (oil-in-water emulsion) in Example 18 had excellent long-term stability and no sticky feeling upon use.

### [Example 19: daytime beauty essence (oil-in-water emulsion)]

An oil-in-water emulsion cosmetic (daytime beauty essence) having the following formulation was prepared by the production method described below.

| (Component) | (% by mass) |
|---|---|
| 1. Microparticles of dimethylpolysiloxane (10%-treated) zincoxide | (10 nm) |
| (component (B)) | 5 |
| 2. Polyhydroxystearic acid (note 1) (component (A)) | 1 |
| 3. Glyceryl oleate (component (C)) | 1 |
| 4. Sorbitan sesquioleate | 0.2 |
| 5. Cetyl 2-ethylhexanoate | 4 |
| 6. Ethylhexyl methoxycinnamate | 7 |
| 7. Diethylamino hydroxybenzoyl hexyl benzoate (note 6) | 2 |
| 8. 2,4-Bis{[4-(2-ethylhexyloxy)-2-hydroxy]-phenyl}-6-(4-methoxyphenyl)-1,3,5-triazine (note 5) | 1 |
| 9. Propylene glycol dicaprylate/dicaprate | 5 |
| 10. Niacinamide | 5 |
| 11. Tranexamic acid | 2 |
| 12. (Sodium acrylate/sodium acryloyldimethyl taurate) copolymer(note 7) | 0.5 |
| 13. Xanthan gum | 0.1 |
| 14. Sodium hyaluronate (note 9) | 0.05 |
| 15. Glycerol (component (D)) | 2 |
| 16. Dipropylene glycol (component (D)) | 2 |
| 17. 1,3-Butylene glycol (component (D)) | 2 |
| 18. Vegetable squalane | 0.5 |
| 19. PEG-10 hydrogenated castor oil | 0.1 |
| 20. PEG-80 hydrogenated castor oil | 1.5 |
| 21. PEG-40 hydrogenated castor oil | 0.3 |
| 22. Ethanol | 5 |
| 23. Purified water | remain |

| | |
|---|---|
| (note 9) Hyaluronic Acid FCH-SU (Kikkoman Biochemifa Company) | |

### (Production method)

A: The components (1) to (5) were mixed and dispersed with a roll mill to give a metal oxide dispersion composition (A).
B: The components (6) to (9) were heated at 75°C and mixed, and the composition (A) was further added. The whole was mixed to give a mixture (B).
C: The components (10) to (23) were homogeneously mixed, and the resulting mixture was added to the mixture (B). The whole was emulsified to give a daytime beauty essence (oil-in-water emulsion).

The daytime beauty essence (oil-in-water emulsion) in Example 19 had excellent long-term stability and no sticky feeling upon use.

### [Example 20: daytime beauty essence (oil-in-water emulsion)]

An oil-in-water emulsion cosmetic (daytime beauty essence) having the following formulation was prepared by the production method described below.

| (Component) | (% by mass) |
|---|---|
| 1. Microparticles of dimethylpolysiloxane (10%-treated) zinc oxide (10 nm) (component (B)) | 5 |
| 2. Polyhydroxystearic acid (note 1) (component (A)) | 1 |
| 3. Glyceryl oleate (component (C)) | 1 |
| 4. Sorbitan oleate | 0.2 |
| 5. Glycerol (component (D)) | 0.1 |
| 6. Cetyl 2-ethylhexanoate | 3 |
| 7. Ethylhexyl methoxycinnamate | 7 |
| 8. Diethylamino hydroxybenzoyl hexyl benzoate (note 6) | 2 |
| 9. 2,4-Bis{[4-(2-ethylhexyloxy)-2-hydroxyl-phenyl}-6-(4-methoxyphenyl)-1,3,5-triazine (note 5) | 1 |
| 10. Propylene glycol dicaprylate/dicaprate | 5 |
| 11. Niacinamide | 5 |
| 12. Tranexamic acid | 2 |
| 13. (Sodium acrylate/sodium acryloyldimethyl taurate) copolymer(note 7) | 0.5 |
| 14. Xanthan gum | 0.1 |
| 15. Sodium hyaluronate (note 9) | 0.05 |
| 16. Glycerol (component (D)) | 0.5 |
| 17. Dipropylene glycol (component (D)) | 1 |
| 18. 1,3-Butylene glycol (component (D)) | 0.5 |
| 19. Vegetable squalane | 0.5 |
| 20. Sodium stearate | 1.5 |
| 21. Polyglyceryl-10 stearate | 0.5 |
| 22. Sodium methyl stearoyl taurate | 0.3 |
| 23. Ethanol | 5 |
| 24. Purified water | remain |

### (Production method)

A: The components (1) to (6) were mixed and dispersed with a roll mill to give a metal oxide dispersion composition (A).
B: The components (7) to (10) were mixed, and the composition (A) was further added. The whole was mixed to give a mixture (B).
C: The components (11) to (24) were homogeneously mixed, and the resulting mixture was added to the mixture (B). The whole was emulsified to give a daytime beauty essence (oil-in-water emulsion).

The daytime beauty essence (oil-in-water emulsion) in Example 20 had excellent long-term stability and no sticky feeling upon use.

## Claims

1. A metal oxide dispersion composition comprising components (A) to (C):
(A) a polyhydroxystearic acid;
(B) a silicone-treated metal oxide; and
(C) a glyceryl oleate,
wherein the metal oxide in the component (B) is a metal oxide excluding titanium oxide.

2. The metal oxide dispersion composition according to claim 1, wherein a mass ratio of the component (B) relative to a total content of the component (A) and the component (C), (B)/{(A) + (C)}, is less than 12.

3. The metal oxide dispersion composition according to claim 1 or 2, wherein the component (B) is a dimethylpolysiloxane-treated metal oxide.

4. The metal oxide dispersion composition according to claim 1 or 2, wherein a content of the component (B) is 20 to 80% by mass.

5. The metal oxide dispersion composition according to claim 1 or 2, wherein a mass ratio of the component (B) relative to the component (C), (B)/(C), is 1 to 30.

6. The metal oxide dispersion composition according to claim 1 or 2, wherein a mass ratio of the component (A) relative to the component (C), (A)/(C), is 0.1 to 3.0.

7. The metal oxide dispersion composition according to claim 1 or 2, wherein the metal oxide in the component (B) is zinc oxide.

8. The metal oxide dispersion composition according to claim 1 or 2, further comprising a component (D), a polyhydric alcohol.

9. A cosmetic or a topical skin preparation comprising the metal oxide dispersion composition according to claim 1 or 2.

10. The cosmetic or the topical skin preparation according to claim 9, wherein the cosmetic or the topical skin preparation is an oil-in-water emulsion.

11. The cosmetic or the topical skin preparation according to claim 9, wherein the cosmetic or the topical skin preparation is used for sun protection.

12. A cosmetic or a topical skin preparation comprising components (A) to (C):
(A) a polyhydroxystearic acid;
(B) a silicone-treated metal oxide; and
(C) a glyceryl oleate,
wherein the metal oxide in the component (B) is a metal oxide excluding titanium oxide.
